# EUROPÄISCHE PATENTSCHRIFT

(11) **EP 2 613 736 B1**
(45) Veröffentlichungstag und Bekanntmachung des Hinweises auf die Patenterteilung: **26.11.2014**
(21) Anmeldenummer: 11755012.9
(22) Anmeldetag: 06.09.2011
(51) Int. Cl.: A61F 2/04, A61F 2/18

(54) **STENT UND VERFAHREN ZU DESSEN HERSTELLUNG**
STENT AND METHOD FOR THE PRODUCTION THEREOF
STENT ET SON PROCÉDÉ DE FABRICATION

(30) Priorität: 06.09.2010 DE 102010044438
(43) Veröffentlichungstag der Anmeldung: 17.07.2013
(73) Patentinhaber: Gottfried Wilhelm Leibniz Universität Hannover, 30167 Hannover (DE); Medizinische Hochschule Hannover, 30625 Hannover (DE); Stiftung Tierärztliche Hochschule Hannover, 30559 Hannover (DE)
(72) Erfinder: BACH, Friedrich-Wilhelm, 30916 Isernhagen (DE); SEITZ, Jan Marten, 30173 Hannover (DE); BORMANN, Dirk, 30926 Seelze (DE); LENARZ, Thomas, 30559 Hannover (DE); SCHWAB, Burkhard, 30966 Hemmingen (DE); KRAMER, Sabine, 30173 Hannover (DE); KIETZMANN, Manfred, 30938 Grossburgwedel (DE)
(74) Vertreter: Günther, Constantin
(86) Internationale Anmeldenummer: PCT/EP2011/004472
(87) Internationale Veröffentlichungsnummer: WO 2012/031737

(56) Entgegenhaltungen:
- WO-A2-2007/140079
- WO-A2-2009/079418

## Beschreibung

Die Erfindung betrifft einen Stent zum Einsetzen in ein Hohlorgan eines Lebewesens gemäß den weiteren Merkmalen der Ansprüche 1 und 3. Die Erfindung betrifft ferner ein Verfahren zur Herstellung eines solchen Stents gemäß dem Anspruch 10.

Als Stents bezeichnet man Gefäßstützen, d.h. medizinische Implantate, die in Hohlorgane eingebracht werden, um diese zu stützen und offen zu halten. Der nachfolgend beschriebene erfindungsgemäße Stent eignet sich grundsätzlich zum Einsetzen in beliebige Hohlorgane eines Lebewesens. Besondere Vorteile bietet der erfindungsgemäße Stent bei Anwendung in Körperorganen, die eine Mukosa, d.h. eine Schleimhaut, aufweisen, wie z.B. die Sinus paranasales.

Chronische Nasennebenhöhlenentzündungen (Sinusitis) werden mittlerweile zu den Zivilisationskrankheiten gezählt. Bedingt durch trockene Heizungsluft, den Einsatz von Klimaanlagen oder auch durch einen Mangel an durchblutungsfördernder Bewegung und eine sehr häufige Verwendung von Nasensprays muss vielen Fällen durch eine Operation die erkrankte Schleimhaut entfernt werden. Vielfach müssen auch neue Öffnungen zu den Nasennebenhöhlen geschaffen werden. Nach einer solchen Operation kommt es zu einer Schwellung der Schleimhaut und zu einer Verstopfung und Verlegung der operativ neu geschaffenen Durchgänge. Dem hat man bisher kurzfristig durch Einlage von SilikonKathetern entgegengewirkt. Ein Problem hierbei ist jedoch eine mittel- bis langfristige Stenosierung durch zirkuläre Narbenbildung. Hierfür gibt es bislang keine zufrieden stellende Abhilfe.

So ist z.B. aus der US 5,601,594 ein nasaler Stent bekannt, der aus Silikon gefertigt wird und zur Arretierung an der Einsatzstelle in der Nase zusammengedrückt eingesetzt wird und sich an der Einsatzstelle wieder ausdehnt. Verwendet werden auch Silikonröhrchen. Diese haben aber ebenfalls den Nachteil, dass wenigstens zwei Operationen erforderlich sind (für Einbau sowie Ausbau). Zudem kommt es über einen relativ langen Zeitraum zu einem Gefühl eines unangenehmen Fremdkörpers in der Nase.

Ein weiterer nasaler Stent geht aus der WO 2007/140079 A2 hervor, welches alle Merkmale des Obebegriffs von Anspruch 1 offenbart.

Es kommt hinzu, dass die beschriebenen Lösungen zu spontaner Dislokation neigen, was z.B. zu einem Wandern bis in die Nasenhaupthöhle führen kann.

Der Erfindung liegt daher die Aufgabe zugrunde, einen verbesserten Stent anzugeben, der die genannten Probleme löst, und ein geeignetes Verfahren zur Herstellung eines solchen Stents anzugeben.

Diese Aufgabe wird durch die in den Ansprüchen 1, 3 und 10 genannte Erfindung gelöst. Die Unteransprüche geben vorteilhafte Ausgestaltungen der Erfindung an.

In einer erfindungsgemäßen Ausgestaltung hat der Stent den Vorteil, dass er vollständig bioresorbierbar ist, d.h. sowohl der Grundkörper als auch die Schutzbeschichtung sind im Körper eines Lebewesens abbaubar. Hierdurch kann eine zweite Operation, die bislang für das Entfernen der bisher verwendeten Implantate notwendig war, vermieden werden. Als Material für den Grundkörper kommt insbesondere Magnesium oder eine Magnesiumlegierung in Frage. Solche Materialien sind im Körper eines Lebewesens gut abbaubar. Das bei der Resorption frei werdende Wasserstoffgas kann dabei gut abgeführt werden. Durch den Einsatz des Stents in einem mit der äußeren Umgebung verbundenen Hohlorgan, wie z.B. im Nasenbereich, ergibt sich der weitere Vorteil, dass eine Verbindung zur umgebenden Luft besteht, so dass das Wasserstoffgas besonders einfach abgeführt werden kann. Der hierbei freiwerdende Wasserstoff kann mit der Atemluft nach außen abgeatmet werden.

Besonders vorteilhaft ist die Verwendung einer Magnesium-Neodym-Legierung als Material für den Grundkörper. Hierdurch können gute Umformeigenschaften des Materials bei der Herstellung des Grundkörpers, z.B. ein günstiges Strangpressverhalten, wie auch eine gute Dilatationsfähigkeit im späteren Gebrauch realisiert werden. Die Magnesium-Neodym-Legierung bietet eine Bruchdehnung im Bereich von 30%. Dies erleichtert die Herstellung eines Stents mittels Kaltverformung ohne Defekte. Auch im Hinblick auf das Resorptionsverhalten ist eine Magnesium-Neodym-Legierung günstig, da Neodym gegenüber der Schleimhaut aufbauhemmende Eigenschaften besitzt und ein Wuchern von Nasenschleimhäuten verhindert.

Der Stent ist als länglicher Hohlkörper ausgebildet, dessen Außenseite dazu eingerichtet ist, an dem Hohlorgan zur Anlage zu kommen. Die Innenseite des Stents bildet einen Durchgangskanal. Der Stent kann grundsätzlich Öffnungen in dem länglichen Hohlkörper aufweisen, z.B. eine gitter- oder geflechtartige Struktur. Vorteilhaft ist es, den länglichen Hohlkörper geschlossen auszubilden, z.B. in einer kreiszylindrischen Form. Hierbei können insbesondere beim Einsetzen des Stents in ein eine Mukosa aufweisendes Hohlorgan unerwünschte Wucherungen weitgehend vermieden werden.

Der erfindungsgemäße Stent eignet sich sowohl für Menschen als auch für Tiere.

In einer erfindungsgemäßen Ausgestaltung weist der Stent eine bioresorbierbare Schutzbeschichtung auf. Die Schutzbeschichtung hat grundsätzlich die Funktion, die Resorption des Materials des Grundkörpers zunächst zu verhindem, da der Stent vor der Resorption für eine gewisse Zeit seine hohlkörperstabilisierende Funktion ausüben soll. Durch die Schutzbeschichtung kann die Dauer bis zur vollständigen Resorption des Stents auf einen gewünschten Wert festgelegt werden, insbesondere durch die Wahl der Dicke der Schutzbeschichtung. Gemäß der Erfindung ist die bioresorbierbare Schutzbeschichtung an der Außenseite des Stents dicker als an der Innenseite des Stents. Hierdurch wird im Verlaufe der Bioresorption zunächst die Schutzbeschichtung an der Innenseite resorbiert, während an der Außenseite noch ein Rest an Schutzbeschichtung verbleibt. Soweit die Schutzbeschichtung an der Innenseite vollständig resorbiert ist, wird der Grundkörper des Stents von seiner Innenseite her resorbiert. Hierdurch kann eine gezielte Degradation des Grundkörpers von seiner Innenseite her realisiert werden. Dies hat den Vorteil, dass die Degradation des Stent-Grundkörpers nicht im Berührungsbereich der Außenseite mit dem Hohlorgan stattfindet, sondern an der Innenseite, was wesentlich schonender für das Lebewesen ist. Insbesondere erfolgt an den empfindlichen Schleimhäuten so gut wie keine Degradation. Der bei der Degradation freiwerdende Wasserstoff kann auf der Innenseite des Stents abgeatmet werden und erzeugt so ebenfalls nahezu keine störenden Einflüsse auf die Schleimhäute.

Als bioresorbierbare Schutzbeschichtung hat sich insbesondere eine Schutzbeschichtung aus Fluorid oder einem ein Fluorid enthaltenden Material herausgestellt. Bei Verwendung eines Grundkörpers, der Magnesium enthält, kann als Schutzbeschichtung insbesondere MgF₂ verwendet werden.

Vorteilhaft ist es, die bioresorbierbare Schutzbeschichtung an der Außenseite des Stents wenigstens doppelt so dick auszubilden wie an der Innenseite des Stents. Gemäß einer vorteilhaften Weiterbildung ist die Schutzbeschichtung an der Außenseite des Stents daher zumindest in etwa doppelt so dick wie an der Innenseite des Stents. Die Schutzbeschichtung kann z.B. eine Dicke 2 µm an der Innenseite und 5 µm an der Außenseite aufweisen.

In einer erfindungsgemäßen Ausgestaltung kann der Stent einen länglichen rohrförmigen Abschnitt und einen an wenigstens einem Ende des rohrförmigen Abschnitts angeordneten, durch einen Katheter dilatierbaren Fixierungsbereich aufweisen. Der Fixierungsbereich ist dazu eingerichtet, den Stent in einem Hohlorgan eines Lebewesens zu fixieren. Vorteilhaft kann der Stent auch an beiden Enden des länglichen rohrförmigen Abschnitts jeweils einen Fixierungsbereich aufweisen.

Der Fixierungsbereich weist eine Mehrzahl von durch einen Katheter dilatierbaren Flügeln auf. Zwei, mehrere oder alle Flügel weisen jeweils eine Flügelfahne auf, die über einen jeweiligen Verbindungssteg mit dem rohrförmigen Abschnitt verbunden ist. Hierbei weist die Flügelfahne in Umfangsrichtung eine größere Erstreckung auf als der Verbindungssteg. Die Flügelfahne ist unsymmetrisch bezüglich einer in Längsrichtung des Stents verlaufenden Mittellinie des Verbindungsstegs, d.h. die Flügelfahne steht an einer Seite des Verbindungsstegs in Umfangsrichtung weiter von dem Verbindungssteg ab als auf der anderen Seite. Möglich ist auch, dass die Flügelfahne an einer Seite in Umfangsrichtung nicht über den Verbindungssteg hinaus absteht. Des Weiteren weisen die unsymmetrischen Flügelfahnen in Umfangsrichtung die gleiche Ausrichtung auf, d.h. die jeweils längeren Überstände der Flügelfahnen weisen in Umfangsrichtung in dieselbe Richtung.

Die dilatierbaren Flügel erlauben eine einfache und sichere Fixierung des Stents in dem Hohlorgan. Die Flügel können auf einfache Weise mit einem Katheter aufgeweitet werden. Vorteilhaft ist der rohrförmige Abschnitt derart steif ausgebildet, dass er über den Katheter nicht dilatierbar ist. Der vorgesehene, hinsichtlich seiner Breite schmaler als die Flügelfahne ausgebildete Verbindungssteg erlaubt dabei ein vereinfachtes Aufweiten der Flügel mit einer relativ geringen Dilatationskraft. Der Verbindungssteg kann je nach Werkstoff relativ dünn ausgebildet sein. Vorteilhaft ist der Verbindungssteg in Umfangsrichtung wenigstens so breit wie die Materialstärke des rohrförmigen Abschnitts. Für eine sinnvolle Auslegung der Abmaße des Verbindungsstegs ist das Verhältnis zwischen Biegeradius und Dilatationskraft zu betrachten, da hiervon die entstehenden Rissneigungen des Materials abhängen. Durch eine Verringerung des Querschnitts des Verbindungsstegs erhöht sich die Rissneigung, gleichzeitig sinkt aber die aufzubringende Dilatationskraft.

Die unsymmetrischen, jedoch in die gleiche Richtung ausgerichteten Flügelfahnen haben sich als besonders vorteilhaft herausgestellt. Durch den auf die Flügel einwirkenden Dilatationsdruck, der z. B. mit einem Ballonkatheter erzeugt wird, wird mittels der mit unsymmetrischen Flügelfahnen versehenen Flügel geometiebedingt neben einer Biegebewegung in Aufweitungsrichtung zusätzlich eine Torsionsbewegung in dem Verbindungssteg erzeugt, die beim Aufweiten auch eine torsionale Bewegung des Flügels ermöglicht. Bei einer solchen Flügelgeometrie wirkt der jeweils größere Überstand der Flügelfahne wie ein Hebel, der eine Torsion des Verbindungsstegs bei der Dilatation durch den Katheter bewirkt. Hierdurch kann die durch die vom Katheter aufgebrachte Kraft besonders effizient in Verformungsenergie umgewandelt werden. Im Ergebnis lässt sich hierdurch ein besonders leichtes, kraftarmes Aufweiten des dilatierbaren Fixierungsbereichs realisieren, das über die einfache Biegebewegung hinausgeht, wobei sich gezeigt hat, dass der Stent nach dem Dilatieren der Fixierungsbereiche einen besonders sicheren Halt in dem Hohlorgan hat.

Eine solches Flügel-Design hat auch den Vorteil, dass grundsätzlich eine beliebige Anzahl von Flügeln herstellbar ist, insbesondere sowohl eine gerade wie auch eine ungerade Anzahl.

Eine Platzierung des Stents in einem Hohlorgan eines Lebewesens sei nachfolgend beispielhaft anhand einer Platzierung in einer Nasennebenhöhle erläutert:

Zunächst werden mittels eines Ballonkatheters ein Nebenhöhlenführungskatheter und ein flexibler Führungsdraht durch den externen Zugang in die Nasenhaupthöhle eingeführt. Über den liegenden Führungsdraht wird der Ballonkatheter wie auf einer Schiene in das Ostium vorgeschoben. Der Ballonkatheter wird genau auf die Höhe des Stirnhöhlenostiums vorgeschoben und aufgedehnt. Hierbei werden zugleich die Fixierungsbereiche auf beiden Seiten des rohrförmigen Abschnitts aufgedehnt, sodann kann der Katheter entfernt werden.

Gemäß einer vorteilhaften Weiterbildung der Erfindung geht eine Seitenwand des Verbindungsstegs im Wesentlichen geradlinig in eine Seitenwand der Flügelfahne über. Als Seitenwände werden die in Umfangsrichtung in den Aussparungen zwischen den Flügeln gebildeten Wände bezeichnet.

Gemäß einer weiteren vorteilhaften Weiterbildung der Erfindung ist der Übergang einer Seitenwand des Verbindungsstegs zu einer Seitenwand der Flügelfahne sprunghaft. Es ergibt sich somit eine Stelle einer sprunghaften Breitenveränderung an dem Übergang von dem Verbindungssteg zu der Flügelfahne. Vorteilhaft ist auch eine Kombination, bei der eine Seitenwand des Verbindungsstegs im Wesentlichen geradlinig in eine Seitenwand der Flügelfahne übergeht und die andere, gegenüberliegende Seitenwand des Verbindungsstegs sprunghaft zu einer Seitenwand der Flügelfahne übergeht.

Gemäß einer vorteilhaften Weiterbildung der Erfindung weist eine zwischen zwei Flügeln gebildete Aussparung am Übergang zum rohrförmigen Abschnitt eine Bohrung mit einem Durchmesser auf, der größer ist als die Breite der Aussparung. Dies erlaubt mit einfachen Mitteln, insbesondere mit einer einfachen Herstellung durch einen Bohrvorgang, den Übergang der Flügel zu dem rohrförmigen Abschnitt dünner zu gestalten als den eigentlichen Flügel, was ebenfalls einer leichteren Verformbarkeit bei der Dilatation des Fixierungsbereichs zugute kommt. Zudem kann durch die hierbei entstehende Abrundung im Übergangsbereich die Rissneigung in diesem Bereich verringert werden und Spannungsspitzen abgebaut werden.

Der Stent weist zwischen zwei benachbarten Flügeln jeweils eine Aussparung auf. Die Aussparung kann z.B. durch Fräsen, Lasern oder Wasserstrahlschneiden hergestellt werden. Die Aussparung kann in einer Grundform eine einfache gerade Form, z.B. eine I-Form, aufweisen. Gemäß einer vorteilhaften Weiterbildung der Erfindung weist wenigstens eine zwischen zwei Flügeln gebildete Aussparung eine L-Form auf. Es hat sich gezeigt, dass das L-förmige Profil besonders vorteilhaft ist, weil hiermit auf fertigungstechnisch einfache Weise eine Mehrzahl von unsymmetrischen und in Umfangsrichtung gleich gerichteten Flügelfahnen hergestellt werden kann.

Gemäß einer vorteilhaften Weiterbildung der Erfindung ist in wenigstens einem Flügel eine Bohrung angeordnet. Die Bohrung ist vorteilhaft ist insbesondere im Übergangsbereich zum rohrförmigen Abschnitt angeordnet. Sofern es sich um einen Flügel mit einem Verbindungssteg handelt, kann die Bohrung auch im Bereich des Verbindungsstegs angeordnet sein. Hierdurch kann die Verformbarkeit des Flügels weiter verbessert werden, ohne die Tendenz zur Rissneigung zu verschlechtern.

Die zuvor erläuterten Ausgestaltungen der Erfindung sind sowohl einzeln als auch in jeglicher Kombination vorteilhaft anwendbar. Insbesondere können je nach Anwendungsgebiet die Fixierungsbereiche auch mit einer Kombination von Flügeln mit und ohne Flügelfahne ausgebildet sein. Vorteilhaft ist es auch, sämtliche Flügel des Fixierungsbereichs mit Flügelfahne auszubilden.

Ein vorteilhaftes Verfahren zur Herstellung eines Stents zum Einsetzen in ein Hohlorgan eines Lebewesens sieht vor, dass ein Grundkörpers des Stents aus einem bioresorbierbaren Material hergestellt wird. Auf die Oberfläche des Grundkörpers wird eine Schutzbeschichtung aus einem ebenfalls bioresorbierbaren Material aufgebracht. Hierbei wird der Stent als länglicher Hohlkörper hergestellt, dessen Außenseite dazu eingerichtet ist, an dem Hohlorgan zur Anlage zu kommen, und an dessen Innenseite ein Durchgangskanal gebildet wird. Weiterhin sind folgende Schritte vorgesehen:
a) Beschichten der Innenseite des Stents mit der bioresorbierbaren Schutzbeschichtung mit einer ersten Dicke und
b) Beschichten der Außenseite des Stents mit der bioresorbierbaren Schutzbeschichtung mit einer zweiten Dicke, wobei die zweite Dicke größer ist als die erste Dicke.

Die Herstellung des Grundkörpers kann dabei derart erfolgen, dass zunächst ein bioresorbierbares metallisches Ausgangsmaterial bereitgestellt wird, wie z.B. die eingangs erwähnten Materialien. Sodann erfolgt ein Herstellen eines metallischen Röhrchens durch Strangpressen. Gegebenenfalls kann sich hieran ein auf Maß ziehen des entstandenen Stentrohlings anschließen. Sodann werden an wenigstens einem Ende des entstandenen Stentrohlings, vorteilhaft an beiden Enden, Fixierungsbereiche hergestellt, indem die Aussparungen durch Einfräsen, Lasern, Wasserstrahlschneiden oder einen anderen geeigneten Herstellvorgang erzeugt werden. Hierdurch entstehen die Flügel und ggf., sofern Flügelfahnen vorgesehen sind, die Flügelfahnen der Fixierungsbereiche. Hieran schließt sich die Schutzbeschichtung aus dem bioresorbierbaren Material an. Für die Schutzbeschichtung können die eingangs genannten Materialien verwendet werden. Zusätzlich kann eine medikamentöse Beschichtung aufgebracht werden.

Gemäß einer vorteilhaften Weiterbildung der Erfindung wird zur Herstellung der bioresorbierbaren Schutzbeschichtung der Stent wenigstens zweimal in ein Gefäß mit einer die Schutzbeschichtung enthaltenden oder erzeugenden Flüssigkeit getaucht. Hierbei wird bei wenigstens einem Tauchvorgang ein die Flüssigkeit zurückhaltender Kern im Innenraum des Stents angeordnet. Durch den Kern wird die Bildung der Schutzbeschichtung an der Innenseite des Stents unterdrückt. Die Flüssigkeit kann entweder direkt das Schutzbeschichtungsmaterial enthalten oder einen anderen geeigneten Stoff, durch den sich beim Eintauchen des Stents durch chemische Reaktion die Schutzbeschichtung bildet. Vorteilhaft kann als Flüssigkeit z.B. Flusssäure verwendet werden. Hierdurch kann eine Fluoridbeschichtung hergestellt werden. Vorteilhaft kann der Kern aus einem Material bestehen, das mit der Flüssigkeit nicht reaktiv ist.

Gemäß einer vorteilhaften Weiterbildung der Erfindung ist bei dem ersten Tauchvorgang der Kern im Innenraum des Stents angeordnet.

Gemäß einer vorteilhaften Weiterbildung der Erfindung kann das Verfahren zur Herstellung des Stents insbesondere folgende Schritte aufweisen:
a) Bereitstellen eines rohrförmigen Stenthalbzeugs,
b) Einlagern des Stenthalbzeugs in NaOH,
c) Einbringen eines mit Flusssäure nicht reaktiven Kerns in das Stenthalbzeug,
d) Tauchen des Stenthalbzeugs in Flusssäure mit damit einhergehender Fluoridisierung,
e) Herausnehmen des nun außen beschichteten Stents,
f) Entfernen des Kerns aus dem Stent,
g) Tauchen des Stents wiederum in die Flusssäure mit damit einhergehender Fluoridisierung sowohl an der Außenseite als auch an der Innenseite,
h) ggf. Beschichten des Stents mit einem Medikament.

Durch das Einführen eines solchen Kerns entsteht an der Außenseite eine dickere Schutzbeschichtung als an der Innenseite, die bei darin angeordnetem Kern so gut wie gar nicht mit der Flusssäure reagiert. Entfernt man den Kern und legt den Stent erneut in Flusssäure, wird die bisher nicht fluoridisierte Innenseite beschichtet und die bereits beschichtete Außenseite zusätzlich beschichtet, mit der Folge, dass hierdurch der Stent außen dicker beschichtet als innen. Bei der späteren Degradation des Stents durch Resorption in einem Hohlorgan eines Lebewesens wird der Stent von innen nach außen abgebaut. Am Ende hat sich der Stent vollständig per Sekretion aufgelöst.

Die Erfindung wird nachfolgend anhand von Ausführungsbeispielen unter Verwendung von Zeichnungen näher erläutert.

Es zeigen
- Figuren 1 und 2: eine erste Ausführung eines Stents und
- Figur 3: den Stent gemäß Figuren 1 und 2 vor und nach dem Dilatieren und
- Figuren 4 bis 6: eine zweite Ausführungsform eines Stents und
- Figuren 7 bis 9: eine dritte Ausführungsform eines Stents und
- Figuren 10 und 11: eine vierte Ausführungsform eines Stents und
- Figuren 12 bis 14: verschiedene Ausführungsformen von mit Flügelfahnen versehenen Flügeln.

In den Figuren werden für einander entsprechende Elemente gleiche Bezugszeichen verwendet.

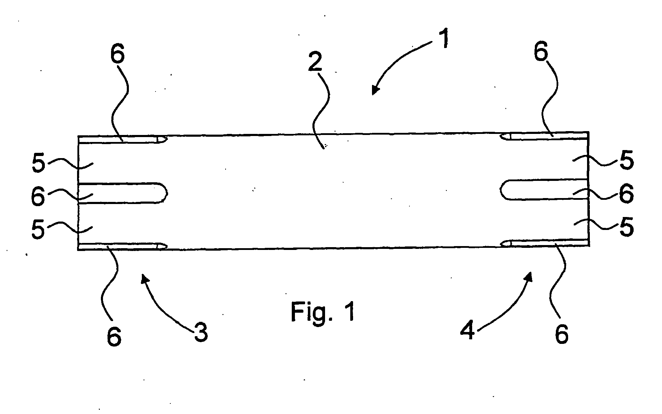

Die Figur 1 zeigt einen Stent 1 in Seitenansicht. Der Stent 1 ist zum Einsetzen in ein Hohlorgan eines Lebewesens eingerichtet. Wie erkennbar ist, ist der Stent 1 grundsätzlich als länglicher Hohlkörper ausgebildet, der eine Außenseite 7 und eine Innenseite 8 aufweist. Im Inneren des länglichen Hohlkörpers wird ein Durchgangskanal 9 gebildet. Der rohrförmige Abschnitt 2 ist vorteilhaft geschlossen ausgebildet, d.h. ohne jegliche Öffnungen.

Der Stent 1 weist einen Grundkörper auf, der im mittleren Bereich einen länglichen rohrförmigen Abschnitt 2 aufweist. An beiden Enden des rohrförmigen Abschnitts 2 schließen sich Fixierungsbereiche 3, 4 an, die derart ausgebildet sind, dass sie zum Zwecke der Fixierung des Stents 1 in dem Hohlorgan durch einen Katheter dilatierbar sind. Hierzu weist jeder Fixierungsbereich 3, 4 eine Mehrzahl von Flügeln 5 auf. Zwischen den Flügeln 5 sind Aussparungen 6 vorgesehen, die Zwischenräume zwischen den Flügeln 5 bilden.

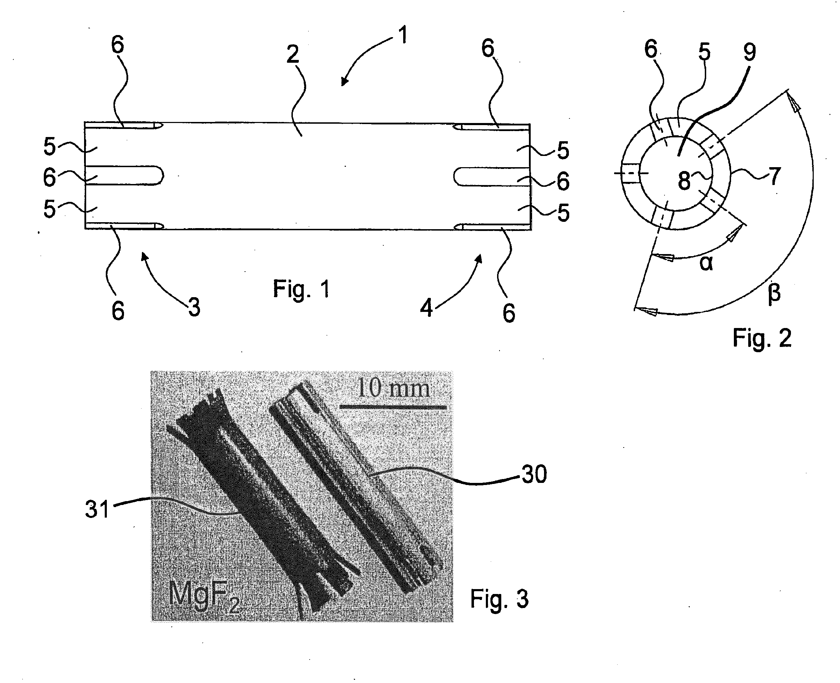

Die Figur 2 zeigt den Stent 1 in einer Draufsicht auf einen Endbereich, so dass die Anordnung der Flügel 5 und der Aussparungen 6 in Umfangsrichtung erkennbar wird.

Wie in den Figuren 1 und 2 erkennbar ist, weist jeder Fixierungsbereich 3, 4 eine Anzahl von fünf Flügeln 5. Alle Flügel 5 weisen gleiche Maße auf, d.h. die Winkelabstände zwischen zwei benachbarten Aussparungen weisen jeweils den Wert α = 72° auf. Entsprechend ergibt sich ein Wert β = 144°.

Der Stent 1 ist aus einem bioresorbierbaren Material hergestellt, insbesondere aus Magnesium oder einer Magnesium enthaltende Legierung, insbesondere aus einer Magnesium-Neodym-Legierung. Des Weiteren ist der Stent 1 auf der Oberfläche des Grundkörpers 2, 3, 4 mit einer die Resorption des Grundkörpers vorübergehend verhindernden Schutzbeschichtung versehen. Die Schutzbeschichtung ist relativ dünn, z.B. 2 µm an der Innenseite 8 und 5 µm an der Außenseite 7. Aufgrund der geringen Schichtdicke ist eine zeichnerische Darstellung der Schutzbeschichtung nicht sinnvoll.

Die Figur 3 zeigt beispielhaft rechts einen noch nicht mit der Schutzbeschichtung versehenen Stent 30 der anhand der Figuren 1 und 2 erläuterten Art. Weiterhin ist links daneben ein mit der Schutzbeschichtung versehener Stent 31 dargestellt. Bei dem Stent 31 wurde zudem bereits eine Dilatation der Fixierungsbereiche 3, 4 vorgenommen. Die in der Figur 3 dargestellte Größenangabe macht beispielhaft die Dimensionen des erfindungsgemäßen Stents klar, wobei die angegebenen Dimensionen für eine Einwendung im tiermedizinischen Bereich ausgelegt sind, z.B. für ein Schwein. Für Anwendungen im humanmedizinischen Bereich kann der Stent entsprechend kleiner ausgebildet werden.

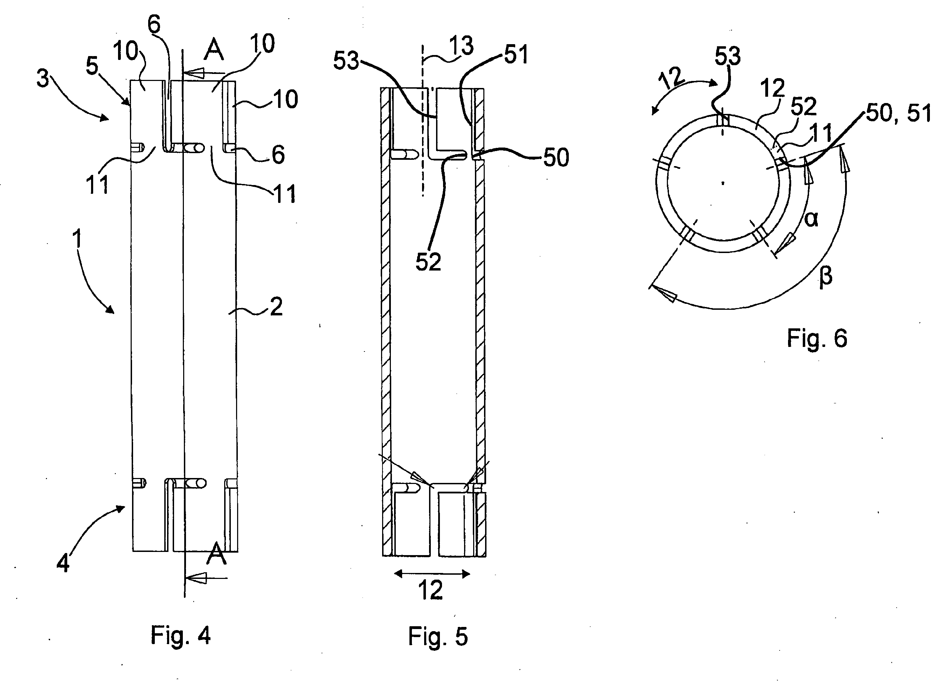

Die Figuren 4 bis 6 zeigen eine zweite Ausführungsform des Stents 1. Die Figur 4 zeigt den Stent 1 in einer Seitenansicht, die Figur 5 zeigt den Stent 1 ebenfalls in Seitenansicht, aber entlang einer Achse A-A geschnitten, die Figur 6 zeigt den Stent in einer Draufsicht auf einen der Fixierungsbereiche 3, 4. Wie erkennbar ist, ist der Stent 1 gemäß den Figuren 4 bis 6 mit einer Mehrzahl von Flügeln 5 in den Fixierungsbereichen 3, 4 versehen, bei denen die Flügel 5 jeweils Flügelfahnen 10 aufweisen, die über jeweilige Verbindungsstege 11 mit dem rohrförmigen Abschnitt 2 verbunden sind. Die Flügelfahnen 10 sowie die Verbindungsstege 11 können z.B. dadurch hergestellt werden, dass zunächst der Stent als rohrförmiges Teil durchgehend ohne Aussparungen hergestellt wird und sodann die Aussparungen 6 in den jeweiligen Fixierungsabschnitten 3, 4 eingefräst werden, so dass sich Aussparungen 6 in L-Form 54 ergeben. Hierbei wird der Fräser zunächst in Längsrichtung des rohrförmigen Teils eingeführt und dann etwa rechtwinklig in Umfangsrichtung 12 des rohrförmigen Teils geführt. Hierdurch ergibt sich die L-förmige Aussparung 54 mit einer am Ende der Aussparung entstehenden Rundung 55.

Die Figur 6 zeigt insbesondere den Verlauf der Flügelfahnen 10 in Umfangsrichtung 12. Wie zudem in den Figuren 5 und 6 dargestellt, ist die Flügelfahne 10 in der Art unsymmetrisch zu einer in Längsrichtung des Stents verlaufenden Mittellinie 13 eines Verbindungsstegs 11 angeordnet, dass an der einen Seite eine Seitenwand 50 des Verbindungsstegs im Wesentlichen gradlinig und ohne Absatz in eine Seitenwand 51 der Flügelfahne 10 übergeht. Auf der anderen Seite ist ein sprunghafter Übergang der gegenüberliegenden Seitenwand 52 des Verbindungsstegs 11 in die entsprechende Seitenwand 53 der Flügelfahne 10 vorgesehen.

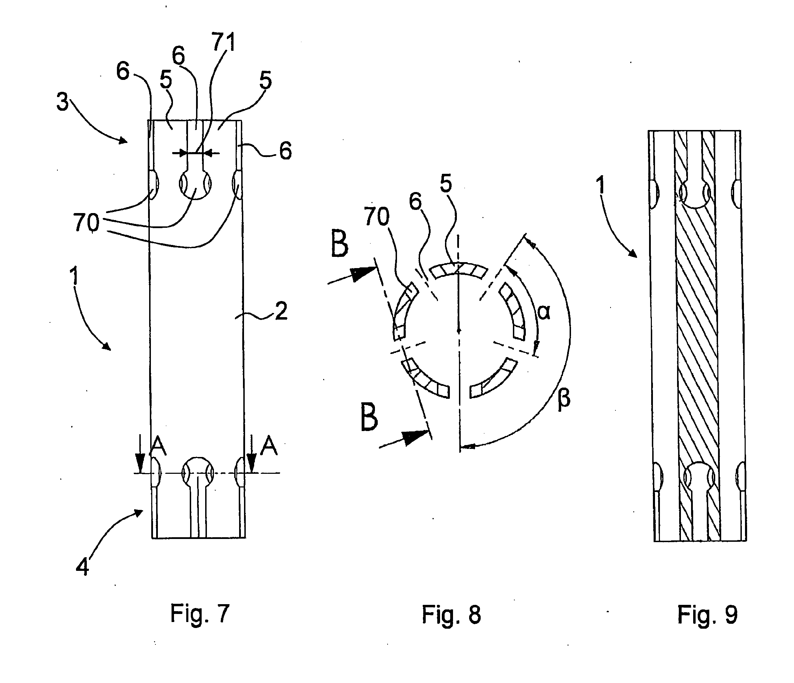

Die Figuren 7 bis 9 zeigen eine dritte Ausführungsform des Stents 1. Hierbei zeigt die Figur 7 den Stent wiederum in einer seitlichen Ansicht, die Figur 8 den Stent in einer Schnittansicht A-A, und die Figur 9 den Stent in einer Seitenansicht mit einem Teilschnitt B-B. Der Stent 1 gemäß den Figuren 7 bis 9 weist Bohrungen 70 auf, die jeweils am Endpunkt einer Aussparung 6 vorgesehen ist, d.h. am Übergang eines Flügels 5 zu dem rohrförmigen Abschnitt 2. Durch die Bohrungen 70, die einen größeren Durchmesser aufweisen als die Breite 71 einer Aussparung 6, kann eine unerwünschte Rissneigung an den Flügeln 5 minimiert werden.

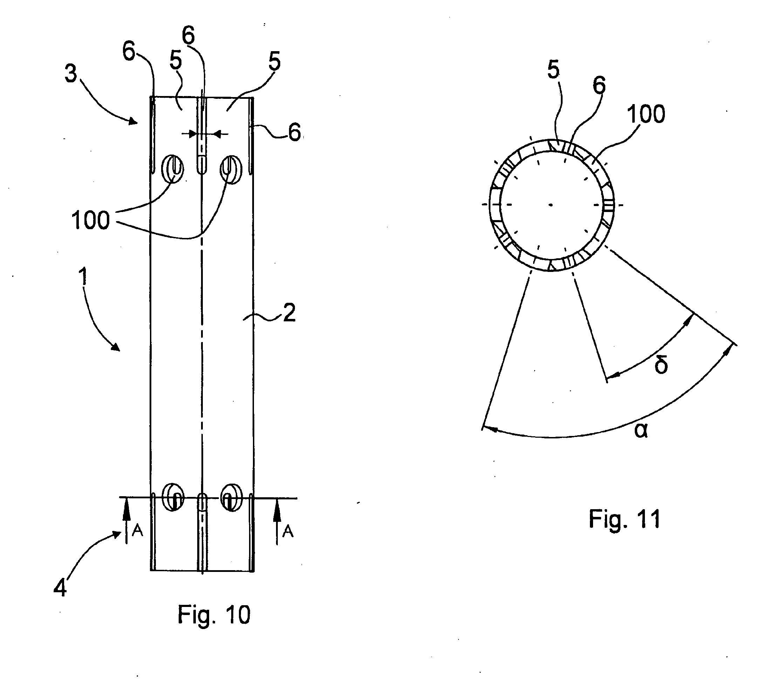

Die Figuren 10 und 11 zeigen eine vierte Ausführungsform des Stents. Hierbei zeigt die Figur 10 den Stent 1 in einer seitlichen Ansicht und die Figur 11 den Stent in einer Schnittdarstellung A-A. Gemäß den Figuren 10 und 11 weist der Stent 1 Bohrungen 100 im Übergangsbereich der Flügel 5 zu dem rohrförmigen Abschnitt 2 auf. Die Bohrungen 100 sind vorteilhaft gleichmäßig in Umfangsrichtung 12 etwa in der Mitte der Flügel 5 angeordnet. Der Winkel α beträgt 72°, der Winkel δ beträgt 36°.

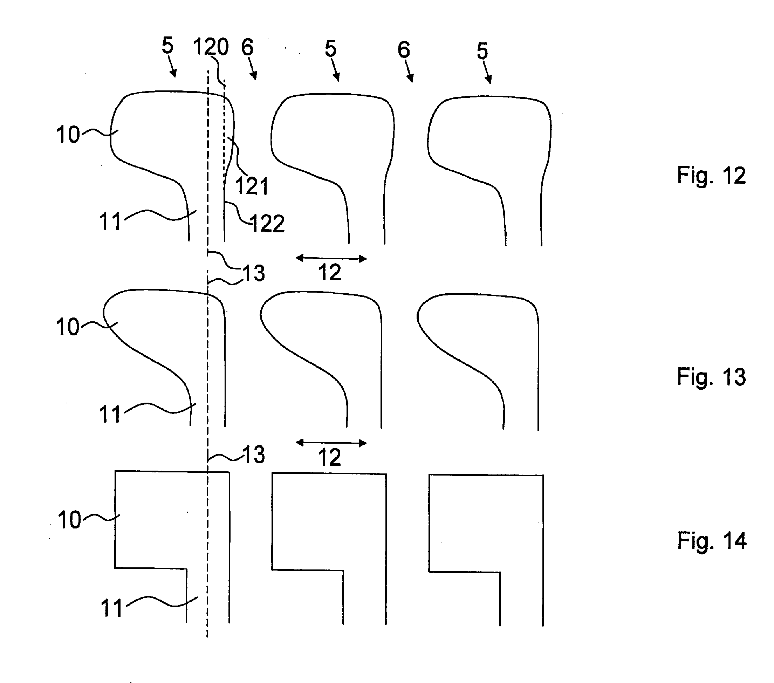

Die Figuren 12 bis 14 zeigen beispielhaft verschiedene vorteilhafte Ausführungsformen von Flügeln 5, die mit unsymmetrischen Flügelfahnen 10 versehen sind. Dargestellt sind in jeder der Figuren 12 bis 14 jeweils drei Flügel 5 mit dazwischen liegenden Aussparungen 6.

Gemäß Figur 12 ist die unsymmetrisch zur Mittellinie 13 des Verbindungsstegs angeordnete Flügelfahne 10 im Wesentlichen mit abgerundeten Eckbereichen versehen. Zudem weist der Flügel 10 auch rechts von der Mittellinie 13 einen Überstand 121 auf. In diesem Bereich steht die Flügelfahne 10 über die Seitenwand 122 des Verbindungsstegs 11 über. Zur Kenntlichmachung ist durch die gestrichelte Linie 120 der theoretische Verlauf bei gradliniger Seitenwand dargestellt.

Die Figur 13 zeigt eine weitere Ausführungsform der Flügelfahnen 10 mit einem links von der Mittellinie 13 stark abgerundeten Design. Rechts von der Mittellinie 13 geht die Seitenwand des Verbindungsstegs 11 linear in eine Seitenwand der Flügelfahne 10 über.

Die Figur 14 zeigt ein im Wesentlichen eckiges Design der Flügelfahne 10. Hierbei geht ebenfalls die rechts von der Mittellinie 13 verlaufende Seitenwand des Verbindungsstegs 11 gradlinig in eine Seitenwand der Flügelfahne 10 über.

## Patentansprüche

1. Stent (1) zum Einsetzen in ein Hohlorgan eines Lebewesens, und zwar ein Hohlorgan, das mit der das Lebewesen umgebenden Luft verbunden ist, wobei der Stent (1) einen aus einem bioresorbierbaren Material hergestellten Grundkörper (2, 3, 4) und eine auf die Oberfläche des Grundkörpers (2, 3, 4) aufgebrachte, die Resorption des Grundkörpers (2, 3, 4) vorübergehend verhindernde Schutzbeschichtung aus einem ebenfalls bioresorbierbaren Material aufweist, wobei der Stent (1) als länglicher Hohlkörper ausgebildet ist, dessen Außenseite (7) dazu eingerichtet ist, an dem Hohlorgan zur Anlage zu kommen, und an dessen Innenseite (8) ein Durchgangskanal (9) gebildet ist, **dadurch gekennzeichnet, dass** die bioresorbierbare Schutzbeschichtung an der Außenseite (7) des Stents (1) eine größere Dicke aufweist als an der Innenseite (8) des Stents (1).

2. Stent nach Anspruch 1, **dadurch gekennzeichnet, dass** die bioresorbierbare Schutzbeschichtung an der Außenseite (7) des Stents (1) wenigstens doppelt so dick ist wie an der Innenseite (8) des Stents (1).

3. Stent (1), nach einem der vorhergehenden Ansprüche, mit einem länglichen rohrförmigen Abschnitt (2) und einem an wenigstens einem Ende des rohrförmigen Abschnitts (2) angeordneten, durch einen Katheter dilatierbaren Fixierungsbereich (3, 4), der dazu eingerichtet ist, den Stent (1) in einem Hohlorgan eines Lebewesens zu fixieren, und zwar in einem Hohlorgan, das mit der das Lebewesen umgebenden Luft verbunden ist, wobei der Fixierungsbereich (3, 4) eine Mehrzahl von durch einen Katheter dilatierbaren Flügeln (5) aufweist, wobei zwei, mehrere oder alle Flügel (5) jeweils eine Flügelfahne (10) aufweist, die über einen jeweiligen Verbindungssteg (11) mit dem rohrförmigen Abschnitt (2) verbunden ist, wobei die Flügelfahne (10) in Umfangsrichtung (12) eine größere Erstreckung aufweist als der Verbindungssteg (11) und die Flügelfahne (10) unsymmetrisch bezüglich einer in Längsrichtung des Stents verlaufenden Mittellinie (13) des Verbindungsstegs (11) ist, wobei die unsymmetrischen Flügelfahnen (10) in Umfangsrichtung (12) die gleiche Ausrichtung aufweisen.

4. Stent nach einem der vorhergehenden Ansprüche, **dadurch gekennzeichnet, dass** der rohrförmige Abschnitt (2) geschlossen ausgebildet ist.

5. Stent nach Anspruch 4, **dadurch gekennzeichnet, dass** eine Seitenwand (50) des Verbindungsstegs (11) im Wesentlichen geradlinig in eine Seitenwand (51) der Flügelfahne (10) übergeht.

6. Stent nach Anspruch 4 oder 5, **dadurch gekennzeichnet, dass** der Übergang einer Seitenwand (52) des Verbindungsstegs (11) zu einer Seitenwand (53) der Flügelfahne (10) sprunghaft ist.

7. Stent nach einem der Ansprüche 4 bis 6, **dadurch gekennzeichnet, dass** eine zwischen zwei Flügeln (5) gebildete Aussparung (6) am Übergang zum rohrförmigen Abschnitt eine Bohrung (70) mit einem Durchmesser aufweist, der größer ist als die Breite (71) der Aussparung (6).

8. Stent nach einem der Ansprüche 4 bis 7, **dadurch gekennzeichnet, dass** wenigstens eine zwischen zwei Flügeln (5) gebildete Aussparung (6) eine L-Form (54) aufweist.

9. Stent nach einem der Ansprüche 4 bis 8, **dadurch gekennzeichnet, dass** in wenigstens einem Flügel (5) eine Bohrung (100) angeordnet ist, insbesondere im Übergangsbereich zum rohrförmigen Abschnitt (2).

10. Verfahren zur Herstellung eines Stents (1) zum Einsetzen in ein Hohlorgan eines Lebewesens, und zwar ein Hohlorgan, das mit der das Lebewesen umgebenden Luft verbunden ist, insbesondere eines Stents (1) nach einem der vorhergehenden Ansprüche, wobei ein Grundkörper (2, 3, 4) des Stents (1) aus einem bioresorbierbaren Material hergestellt wird und auf die Oberfläche des Grundkörpers (2, 3, 4) eine Schutzbeschichtung aus einem ebenfalls bioresorbierbaren Material aufgebracht wird, wobei der Stent (1) als länglicher Hohlkörper hergestellt wird, dessen Außenseite (7) dazu eingerichtet ist, an dem Hohlorgan zur Anlage zu kommen, und an dessen Innenseite (8) ein Durchgangskanal gebildet wird, mit den Schritten:
a) Beschichten der Innenseite (8) des Stents (1) mit der bioresorbierbaren Schutzbeschichtung mit einer ersten Dicke und
b) Beschichten der Außenseite (7) des Stents (1) mit der bioresorbierbaren Schutzbeschichtung mit einer zweiten Dicke, wobei die zweite Dicke größer ist als die erste Dicke.

11. Verfahren nach Anspruch 10, **dadurch gekennzeichnet, dass** der Stent (1) zur Herstellung der bioresorbierbaren Schutzbeschichtung wenigstens zweimal in ein Gefäß mit einer die Schutzbeschichtung enthaltenden oder erzeugenden Flüssigkeit getaucht wird, wobei bei wenigstens einem Tauchvorgang ein die Flüssigkeit zurückhaltender Kern im Innenraum (9) des Stents (1) angeordnet ist.

12. Verfahren nach Anspruch 11, **dadurch gekennzeichnet, dass** bei dem ersten Tauchvorgang der Kern im Innenraum (9) des Stents (1) angeordnet ist.

13. Verfahren nach einem der Ansprüche 10 bis 12, **gekennzeichnet durch** Strangpressen eines metallischen Röhrchens aus einem bioresorbierbaren Ausgangsmaterial zur Erzeugung des Grundkörpers (2, 3, 4) und anschließendes Beschichten des Grundkörpers (2, 3, 4) mit der bioresorbierbaren Schutzbeschichtung.

## Claims

1. Stent (1) for insertion into a hollow organ of a living being, specifically a hollow organ that is connected to the air surrounding the living being, wherein the stent (1) has a main body (2, 3, 4) produced from a bioresorbable material, and a protective coating which is made of a likewise bioresorbable material and which is applied to the surface of the main body (2, 3, 4) and temporarily prevents the resorption of the main body (2, 3, 4), wherein the stent (1) is designed as an elongate hollow body whose outer face (7) is intended to bear on the hollow organ and on whose inner face (8) a continuous channel (9) is formed, **characterized in that** the bioresorbable protective coating on the outer face (7) of the stent (1) has a greater thickness than on the inner face (8) of the stent (1).

2. Stent according to Claim 1, **characterized in that** the bioresorbable protective coating on the outer face (7) of the stent (1) is at least twice as thick as on the inner face (8) of the stent (1).

3. Stent (1), according to one of the preceding claims, with an elongate tubular portion (2) and a fixing area (3, 4) which is arranged at at least one end of the tubular portion (2) and is dilatable by a catheter, said fixing area (3, 4) being intended to fix the stent (1) in a hollow organ of a living being, specifically in a hollow organ that is connected to the air surrounding the living being, wherein the fixing area (3, 4) has a plurality of wings (5) that are dilatable by a catheter, wherein two, several or all of the wings (5) each have a wing tab (10), which is connected to the tubular portion (2) via a respective connecting web (11), wherein the wing tab (10) has a greater extent in the circumferential direction (12) than the connecting web (11), and the wing tab (10) is asymmetrical with respect to a centre line (13) of the connecting web (11) extending in the longitudinal direction of the stent, wherein the asymmetrical wing tabs (10) have the same orientation in the circumferential direction (12).

4. Stent according to one of the preceding claims, **characterized in that** the tubular portion (2) has a closed design.

5. Stent according to Claim 4, **characterized in that** a side wall (50) of the connecting web (11) merges substantially rectilinearly into a side wall (51) of the wing tab (10).

6. Stent according to Claim 4 or 5, **characterized in that** the transition from a side wall (52) of the connecting web (11) to a side wall (53) of the wing tab (10) is abrupt.

7. Stent according to one of Claims 4 to 6, **characterized in that** a cutout (6) formed between two wings (5) has, at the transition to the tubular portion, a bore (70) with a diameter greater than the width (71) of the cutout (6).

8. Stent according to one of Claims 4 to 7, **characterized in that** at least one cutout (6) formed between two wings (5) has an L shape (54).

9. Stent according to one of Claims 4 to 8, **characterized in that** a bore (100) is arranged in at least one wing (5), in particular in the transition area to the tubular portion (2).

10. Method for producing a stent (1) for insertion into a hollow organ of a living being, specifically a hollow organ that is connected to the air surrounding the living being, in particular a stent (1) according to one of the preceding claims, wherein a main body (2, 3, 4) of the stent (1) is produced from a bioresorbable material, and a protective coating which is made of a likewise bioresorbable material is applied to the surface of the main body (2, 3, 4), wherein the stent (1) is produced as an elongate hollow body whose outer face (7) is intended to bear on the hollow organ and on whose inner face (8) a continuous channel is formed, said method having the steps of:
a) coating the inner face (8) of the stent (1) with the bioresorbable protective coating in a first thickness, and
b) coating the outer face (7) of the stent (1) with the bioresorbable protective coating in a second thickness, wherein the second thickness is greater than the first thickness.

11. Method according to Claim 10, **characterized in that**, in order to produce the bioresorbable protective coating, the stent (1) is immersed at least twice into a vessel that holds a liquid containing or generating the protective coating, wherein a core retaining the liquid is arranged in the interior (9) of the stent (1) in at least one immersion process.

12. Method according to Claim 11, **characterized in that** the core is arranged in the interior (9) of the stent (1) in the first immersion process.

13. Method according to one of Claims 10 to 12, **characterized by** extrusion of a metallic tube made of a bioresorbable starting material in order to generate the main body (2, 3, 4), and subsequent coating of the main body (2, 3, 4) with the bioresorbable protective coating.

## Revendications

1. Stent (1) pour l'utilisation dans un organe creux d'un organisme vivant, en particulier un organe creux qui est en communication avec l'air entourant l'organisme vivant, le stent (1) présentant un corps de base (2, 3, 4) fabriqué en un matériau biorésorbable et un revêtement de protection appliqué sur la surface du corps de base (2, 3, 4), empêchant temporairement la résorption du corps de base (2, 3, 4), constitué d'un matériau également biorésorbable, le stent (1) étant réalisé sous forme de corps creux allongé, dont le côté extérieur (7) est prévu pour venir en appui contre l'organe creux, et sur le côté intérieur (8) duquel est formé un canal de passage (9), **caractérisé en ce que** le revêtement de protection biorésorbable sur le côté extérieur (7) du stent (1) présente une plus grande épaisseur que sur le côté intérieur (8) du stent (1).

2. Stent selon la revendication 1, **caractérisé en ce que** le revêtement de protection biorésorbable sur le côté extérieur (7) du stent (1) est au moins deux fois plus épais que sur le côté intérieur (8) du stent (1).

3. Stent (1), selon l'une quelconque des revendications précédentes, comprenant une section allongée de forme tubulaire (2) et une région de fixation (3, 4) disposée à au moins une extrémité de la section de forme tubulaire (2), pouvant être dilatée par un cathéter, qui est prévue pour fixer le stent (1) dans un organe creux d'un organisme vivant, en particulier dans un organe creux qui est en communication avec l'air entourant l'organisme vivant, la région de fixation (3, 4) présentant une pluralité d'ailettes (5) pouvant être dilatées par un cathéter, deux, plusieurs, ou la totalité des ailettes (5) présentant à chaque fois un fanion d'ailette (10) qui est connecté par le biais d'une nervure de connexion respective (11) à la section de forme tubulaire (2), le fanion d'ailette (10) présentant, dans la direction périphérique (12), une plus grande étendue que la nervure de connexion (11) et le fanion d'ailette (10) étant asymétrique par rapport à un axe médian (13) de la nervure de connexion (11) s'étendant dans la direction longitudinale du stent, les fanions d'ailette asymétriques (10) présentant la même orientation dans la direction périphérique (12).

4. Stent selon l'une quelconque des revendications précédentes, **caractérisé en ce que** la section de forme tubulaire (2) est réalisée sous forme fermée.

5. Stent selon la revendication 4, **caractérisé en ce qu'**une paroi latérale (50) de la nervure de connexion (11) se prolonge essentiellement en ligne droite par une paroi latérale (51) du fanion d'ailette (10).

6. Stent selon la revendication 4 ou 5, **caractérisé en ce que** la transition d'une paroi latérale (52) de la nervure de connexion (11) à une paroi latérale (53) du fanion d'ailette (10) est brutale.

7. Stent selon l'une quelconque des revendications 4 à 6, **caractérisé en ce qu'**un évidement (6) formé entre deux ailettes (5) présente, au niveau de la transition à la section de forme tubulaire, un alésage (70) ayant un diamètre supérieur à la largeur (71) de l'évidement (6).

8. Stent selon l'une quelconque des revendications 4 à 7, **caractérisé en ce qu'**au moins un évidement (6) formé entre deux ailettes (5) présente une forme en L (54).

9. Stent selon l'une quelconque des revendications 4 à 8, **caractérisé en ce que** dans au moins une ailette (5) est disposé un alésage (100), en particulier dans la région de transition à la section de forme tubulaire (2).

10. Procédé de fabrication d'un stent (1) pour l'utilisation dans un organe creux d'un organisme vivant, en particulier un organe creux qui est en communication avec l'air entourant l'organisme vivant, en particulier d'un stent (1) selon l'une quelconque des revendications précédentes, dans lequel un corps de base (2, 3, 4) du stent (1) est fabriqué en un matériau biorésorbable et un revêtement de protection en un matériau également biorésorbable est appliqué sur la surface du corps de base (2, 3, 4), le stent (1) étant fabriqué sous forme de corps creux allongé, dont le côté extérieur (7) est prévu pour venir en appui contre l'organe creux, et sur le côté intérieur (8) duquel est formé un canal de passage, comprenant les étapes suivantes :
a) revêtement du côté intérieur (8) du stent (1) avec le revêtement de protection biorésorbable avec une première épaisseur et
b) revêtement du côté extérieur (7) du stent (1) avec le revêtement de protection biorésorbable avec une deuxième épaisseur, la deuxième épaisseur étant supérieure à la première épaisseur.

11. Procédé selon la revendication 10, **caractérisé en ce que** le stent (1), pour la fabrication du revêtement de protection biorésorbable, est immergé au moins deux fois dans un récipient comprenant un liquide contenant ou produisant le revêtement de protection, un noyau retenant le liquide étant disposé dans l'espace intérieur (9) du stent (1) lors d'au moins une opération d'immersion.

12. Procédé selon la revendication 11, **caractérisé en ce que** lors de la première opération d'immersion, le noyau est disposé dans l'espace intérieur (9) du stent (1).

13. Procédé selon l'une quelconque des revendications 10 à 12, **caractérisé par** le filage d'un petit tuyau métallique constitué d'un matériau de départ biorésorbable pour produire le corps de base (2, 3, 4) et le revêtement subséquent du corps de base (2, 3, 4) avec le revêtement de protection biorésorbable.
